# EUROPEAN PATENT SPECIFICATION

(11) **EP 4 251 983 B1**
(45) Date of publication and mention of the grant of the patent: **14.05.2025**
(21) Application number: 22752041.8
(22) Date of filing: 10.02.2022
(51) Int. Cl.: G01N 27/02, A61F 13/42

(54) **MOISTURE DETECTION AND ESTIMATION WITH MULTIPLE FREQUENCIES**
FEUCHTIGKEITSDETEKTION UND -SCHÄTZUNG MIT MEHREREN FREQUENZEN
DÉTECTION ET ESTIMATION D'HUMIDITÉ À L'AIDE DE FRÉQUENCES MULTIPLES

(30) Priority: 11.02.2021 US 202163148440 P
(43) Date of publication of application: 04.10.2023
(73) Proprietor: Tuli, Raja, Montreal, Québec H2X 3R8 (CA)
(72) Inventor: Tuli, Raja, Montreal, Québec H2X 3R8 (CA)
(74) Representative: Loo, Chi Ching
(86) International application number: PCT/CA2022/050199
(87) International publication number: WO 2022/170435

(56) References cited:
- WO-A1-2006/079621
- WO-A1-2016/079621
- US-A- 5 760 694
- US-A1- 2006 033 585
- US-A1- 2015 285 752
- US-A1- 2015 285 752

## Description

### BACKGROUND OF THE INVENTION

### Field of the Invention

The present disclosure relates to moisture detection and estimation in an absorbent article such as diaper.

### Description of Related Art

Disposable absorbent article such as disposable diaper is a product that is capable of receiving and retaining bodily exudates or excretions so as to prevent contamination of the clothing or external environment. As an example, with a disposable diaper, the user is allowed to urinate or defecate without the use of a toilet. In addition to diapers, there are numerous other types of disposable absorbent articles such as e.g. under pads, incontinence pads, fitted briefs, belted shields, liners, all-in-one pads, pullup incontinence pants, training pants, protective underwear, catamenial napkins, and incontinence guards etc. It is to be understood that the list of disposable absorbent articles identified above is not exhaustive and that these and other absorbent articles can be used with the present disclosure and are within the scope of the present disclosure. It is also to be understood that a reference in this specification to any one such article, such as a "diaper" is to be taken to be a reference to any and all other suitable absorbent articles including incontinence garments, pads and the like.

In order to prevent contamination of the clothing or external environment, disposable absorbent article is provided with an absorbent core capable of receiving and retaining bodily exudates or excretions, and a substantially liquid impervious layer. In general, disposable absorbent products consist of a layered construction, which allows the bodily exudates or excretions to be distributed and transferred to the absorbent core where they are retained in. In everyday use, a disposable absorbent article may be used until the absorbent core is saturated with e.g. bodily exudates or excretions. When the absorbent core is saturated, the disposable absorbent article needs to be removed, disposed of, and replaced with a clean and dry article.

Without a solution for moisture detection and estimation of an absorbent article, the user wearing the absorbent article might be left in their own urine and feces for extended periods of time, causing many health problems.

Therefore, there is a need for a solution for moisture detection and estimation of an absorbent article.

The present disclosure addresses this and other prior art shortcoming..

### BRIEF SUMMARY OF THE INVENTION

Embodiments are presented herein of, inter alia, moisture detection and estimation in an absorbent article with use of multiple frequencies.

In an embodiment of the present disclosure, a method of moisture detection and estimation in an absorbent article with use of multiple frequencies is provided that comprises: applying, by capacitive coupling, drive signal of multiple frequencies to a drive electrode in the absorbent article; sensing, by capacitive coupling, sense signal of the multiple frequencies from a sense electrode in the absorbent article; detecting the first wetness event in the absorbent article with use of sense signal of a frequency of the multiple frequencies; and detecting the saturation of the absorbent article with use of sense signal of another frequency of the multiple frequencies.

This summary is intended to provide a brief overview of some of the subject matter described in this document. Accordingly, it will be appreciated that the above-described features are merely examples and should not be construed to narrow the scope or spirit of the subject matter described herein in any way. Other features, aspects, and advantages of the subject matter described herein will become apparent from the following Detailed Description, Figures, and Claims.

### BRIEF DESCRIPTION OF THE DRAWINGS

The various preferred embodiments of the present invention described herein can be better understood by those skilled in the art when the following detailed description is read with reference to the accompanying drawings. The components in the figures are not necessarily drawn to scale and any reference numeral identifying an element in one drawing will represent the same element throughout the drawings. The figures of the drawing are briefly described as follows.
Figure 1 illustrates an exemplary disposable absorbent article in an exploded perspective view, according to an embodiment of the present disclosure.
Figure 2 illustrates an exemplary disposable absorbent article with four spaced-apart conductive lines being provided on the top side of the substantially liquid impervious layer, according to an embodiment of the present disclosure.
Figure 3 illustrates an exemplary pod that may be used with the exemplary disposal absorbent article as illustrated in Figure 2 for moisture detection and estimation of the absorbent article, according to an embodiment of the present disclosure.
Figure 4 illustrates amplitude vs. time graph of an exemplary drive signal Sd according to an embodiment of the present disclosure.
Figure 5 illustrates amplitude vs. time graphs G showing the sense signals Ss of various frequencies, according to an embodiment of the present disclosure.

### DETAILED DESCRIPTION OF THE INVENTION

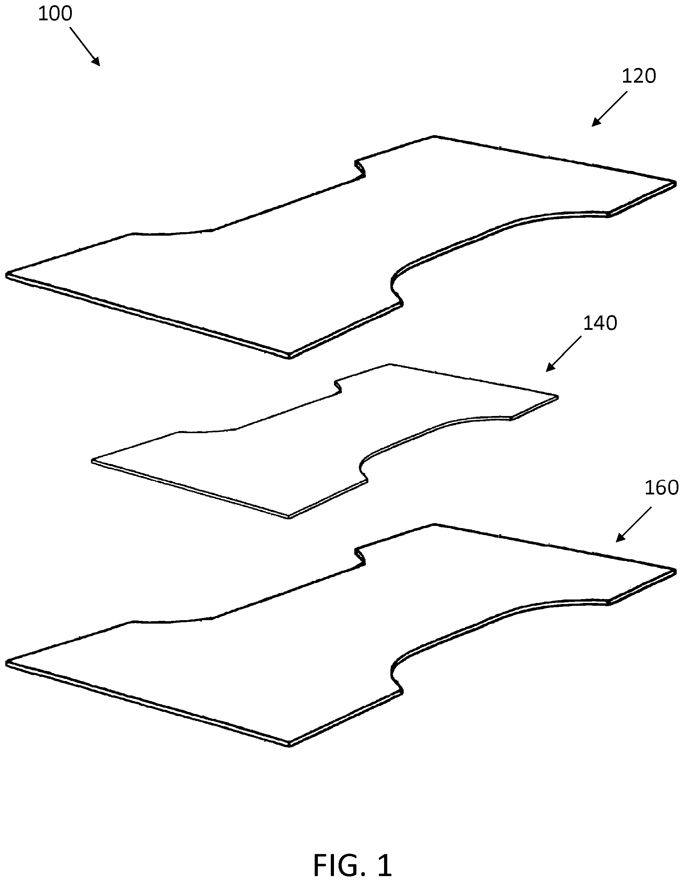

Figure 1 illustrates an exemplary disposable absorbent article in an exploded perspective view. As illustrated, a disposable absorbent article 100 primarily consists of an absorbent core 140 sandwiched between a liquid pervious layer 120 and a substantially liquid impervious layer 160.

As illustrated in the exemplary diaper of Figure 1, a disposable absorbent article 100 has a substantially liquid impervious layer 160 configured to prevent the bodily exudates or excretions absorbed and retained in the absorbent core 140 from wetting articles, such as bed sheets and undergarments, which contact the disposable absorbent article 100. On top of the layer 160 is disposed an absorbent core 140 made of a superabsorbent material. On top of the absorbent core 140 is a liquid pervious layer 120 that is joined to the layer 160 in an assembled state of the disposable absorbent article and is placed next to the skin of the user when in use. Additional structural features such as additional layer(s), elastic members and fastening means for securing the article in place, such as tape tab fasteners, may also be included.

The liquid pervious layer 120 is configured to be penetrable by bodily exudates and excretions in a direction into the absorbent core 140 to enable them to be absorbed and retained in the underlying absorbent core 140. It is appreciated that the layer 120 may be made of a variety of liquid pervious materials, e.g. nonwoven fabric.

The absorbent core 140 is made up of hydrophilic superabsorbent polymers (SAP) and fibrous material, as a non-limiting example. The polymers act like tiny sponges that retain many times their weight in liquid.

The substantially liquid impervious layer 160 is made of a material substantially impervious to liquids. As an example, the substantially liquid impervious layer 160 may be manufactured from a thin plastic film, although other liquid impervious materials may also be used. As described above, the substantially liquid impervious layer 160 is configured to prevent the bodily exudates or excretions absorbed and retained in the absorbent core from wetting articles, such as bed sheets and undergarments, which contact the diaper.

As illustrated in the exemplary diaper of Figure 1, the layers 120 and 160 are coextensive and have generally larger dimension, in length and/or width, than the absorbent core 140.

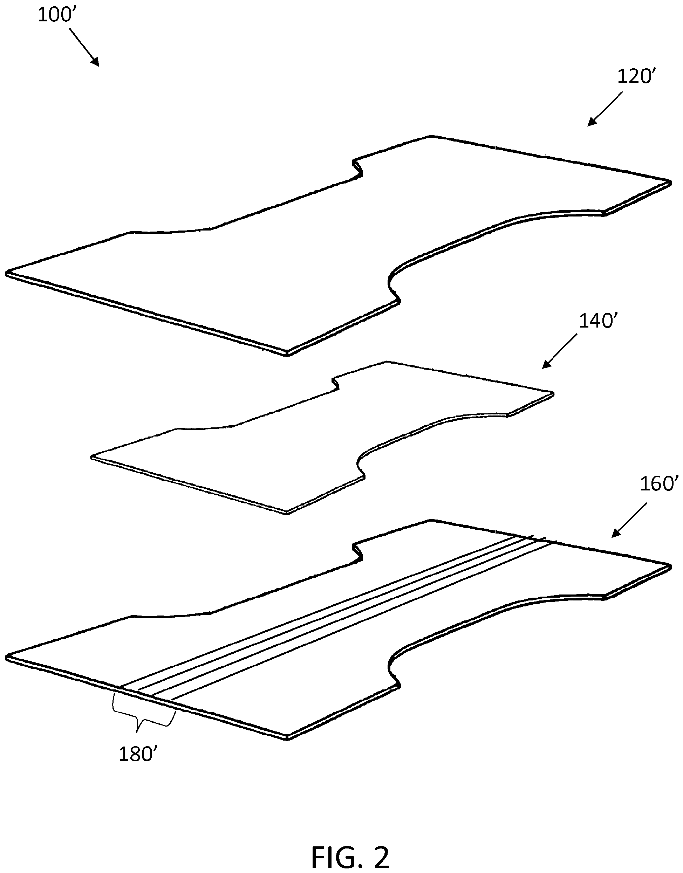

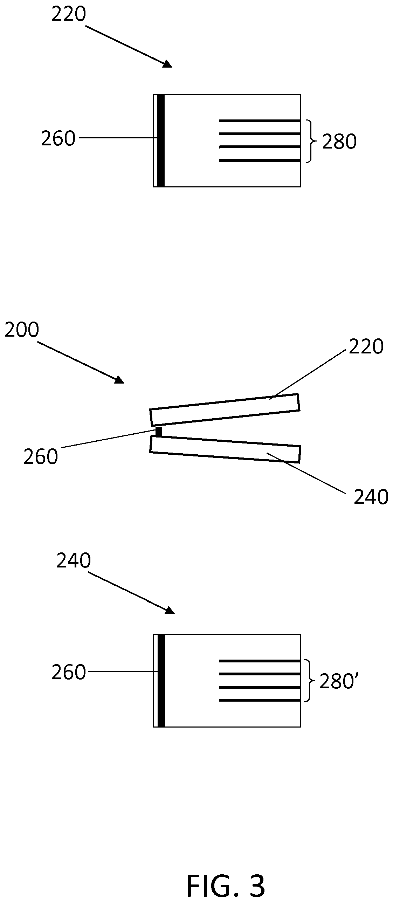

In order for moisture detection and estimation, in particular to detect the presence and/or amount of the bodily exudates or excretions in a disposable absorbent article, in particular in its absorbent core, a number of (e.g. at least two) spaced-apart conductive lines are provided as electrodes on the top side (i.e. the side facing the absorbent core) of the substantially liquid impervious layer along the length of the disposable absorbent article, in an embodiment of the present disclosure. In Figure 2, an exemplary disposable absorbent article 100' is depicted with four spaced-apart conductive lines 180' being provided on the top side of the substantially liquid impervious layer 160', as an example. The spaced-apart conductive lines 180' in the disposable absorbent article 100' operate in cooperation with a pod 200 (to be described below in reference to Figure 3), for moisture detection and estimation of the absorbent article 100'.

As illustrated in Figure 3, an exemplary pod 200 primarily consists of two halves 220 and 240 that are pivotably coupled to each other with a pivotal connection 260. At least one of the two halves 220 and 240 is provided with a number of (e.g. at least two) contacts 280 or 280' on its inner side (i.e. the side facing the other half). As a non-limiting example, both the two halves 220 and 240 have each a number of contacts 280 or 280' on their respective inner side, as illustrated in Figure 3. In the example as illustrated in Figure 3, there are four (the same number as the conductive lines 180' as illustrated in Figure 2) contacts 280 and 280' on the two halves 220 and 240 respectively.

In operation, the pod 200 as illustrated in Figure 3 is clipped on a disposable absorbent article (e.g. 100' as illustrated in Figure 2) at one of its waist end edges and coupled to the conductive lines (e.g. 180' in Figure 2) with the contacts 280 and/or 280' on the pod 200. In use, the bodily exudates or excretions absorbed and retained in the absorbent core (e.g. 140' in Figure 2) of the absorbent article will cause at least two of the spaced-apart electrodes (conductive lines, e.g. 180' in Figure 2) to be connected to each other, and thus the pod 200 can detect the presence and/or amount of the exudates or excretions in the disposal absorbent article (e.g. 100' in Figure 2) by applying a drive signal to at least one of the electrodes (the drive electrode) and sensing a sense signal from at least another one of the electrodes (the sense electrode).

As mentioned above, in a disposable absorbent article the spaced-apart conductive lines may be provided on any layer, on which at least two of the spaced-apart conductive lines, with the aid of the bodily exudates or excretion absorbed and retained in the disposable absorbent article, will connect to each other, which in turn enables the detection of the presence and/or amount of the exudates or excretions in the disposal absorbent article.

It is to be noted that the pod 200, in particular its contacts 280 and 280', cannot make physical contact with the electrodes (conductive lines, e.g. 180' in Figure 2) on the disposable absorbent article (e.g. 100' in Figure 2) due to the presence of outermore material or layer(s) of the article, e.g. material or layer(s) further away from the absorbent core, with respect to the conductive lines, e.g. the substantially liquid impervious layer (e.g. 160' in Figure 2) and/or the liquid pervious layer (e.g. 120' in Figure 2). That is, in use, the pod 200 is capacitively coupled to the electrodes (conductive lines, e.g. 180' in Figure 2) by aligning its contacts 280 and 280' with the conductive lines (e.g. 180' in Figure 2) and placing them in close proximity.

As mentioned above, an absorbent article such as a diaper is provided with at least two electrodes, i.e. at least one drive electrode and at least one sense electrode, in an embodiment of the present disclosure. Further, a pod is provided to make capacitive contact with these electrodes in the absorbent article, that is, the pod touches the electrodes but does not make physic contact with electrodes.

In an embodiment of the present disclosure, in order for moisture detection and estimation, a drive signal is applied to a drive electrode in the absorbent article, and in response, a sense signal is sensed from a sense electrode in the absorbent article. It is appreciated that the drive signal cannot be a DC signal because of the capacitive coupling.

At a first wetness event in an absorbent article a significant rise (e.g. a sharp jump or a gradual upslope) in amplitude presents in a sense signal of a low frequency (e.g. around 10 kHz), which however does not change much either before or after the first wetness event even though further wetness events occur in the absorbent article later.

Further, a sense signal of high frequency e.g. ranging from 70 kHz - 500 Khz gradually increases in its amplitude as more and more wetness events occur in the absorbent article.

Based on the above, multiple frequencies may be used for moisture detection and estimation of an absorbent article in an embodiment of the present disclosure.

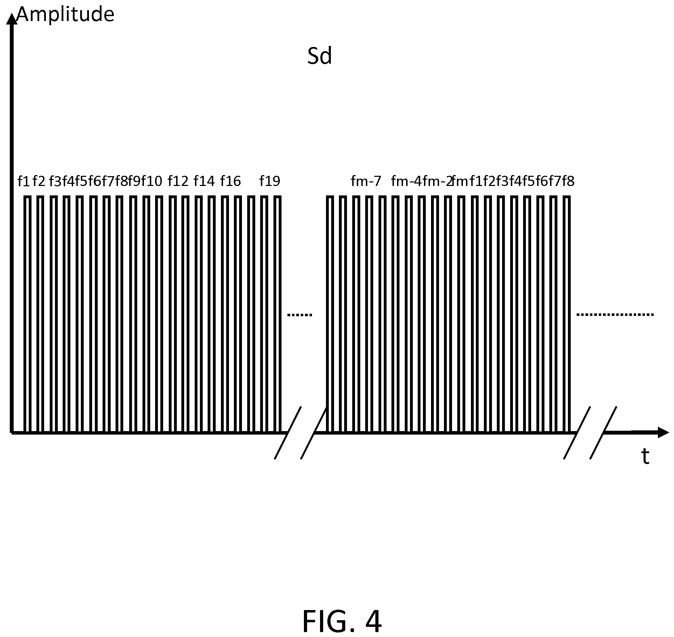

In particular, in an embodiment of the present disclosure the drive signal that is to be applied to the drive electrode of the absorbent article may be a periodic signal that is swept with the same amplitude in a period over a range of frequencies f1, f2, ..., fm, such as e.g. 5 kHz, 10 kHz, ..., 3 MHz. For example, in a period the drive signal Sd is a sinewave signal that is swept over frequencies ranged from f1 to fm such as e.g. 5 kHz to 3 MHz, with each frequency being of the same amplitude and lasting for the same time interval e.g. 10ms. In particular, in a period, the drive signal 100 is a sinewave signal of 5 kHz from 0ms to 10ms, then a sinewave signal of 10 kHz from 10ms to 20 ms, then a sinewave signal of 15 kHz from 20ms to 30ms, ..., finally a sinewave signal of 3 MHz from 1.99s to 2s, and then repeats periodically, as an example. It is appreciated that between different frequencies there may exist time interval with no signal. For example, the drive signal may be a sinewave signal of 5 kHz from 0ms to 10ms, then 0 (i.e. no signal) from 10ms to20ms, then a sinewave signal of 10 kHz from 20ms to 30 ms, then 0 from 30ms to 40ms, then a sinewave signal of 15 kHz from 40ms to 50ms, ..., finally a sinewave signal of 3 MHz from 3.98s to 3.99s, then 0 from 3.99s to 4s, and then repeats periodically, as an example. As an example, Figure 4 illustrates amplitude vs. time graph of an exemplary drive signal Sd according to an embodiment of the present disclosure.

In response to this drive signal Sd being applied to a drive electrode of an absorbent article, a sense signal 200 is sensed from a sense electrode of the absorbent article. It is appreciated that in response to a sinewave signal of a specific frequency being applied to a drive electrode of an absorbent article, a sinewave signal of the same specific frequency is sensed from a sense electrode of the absorbent article, but with a smaller amplitude.

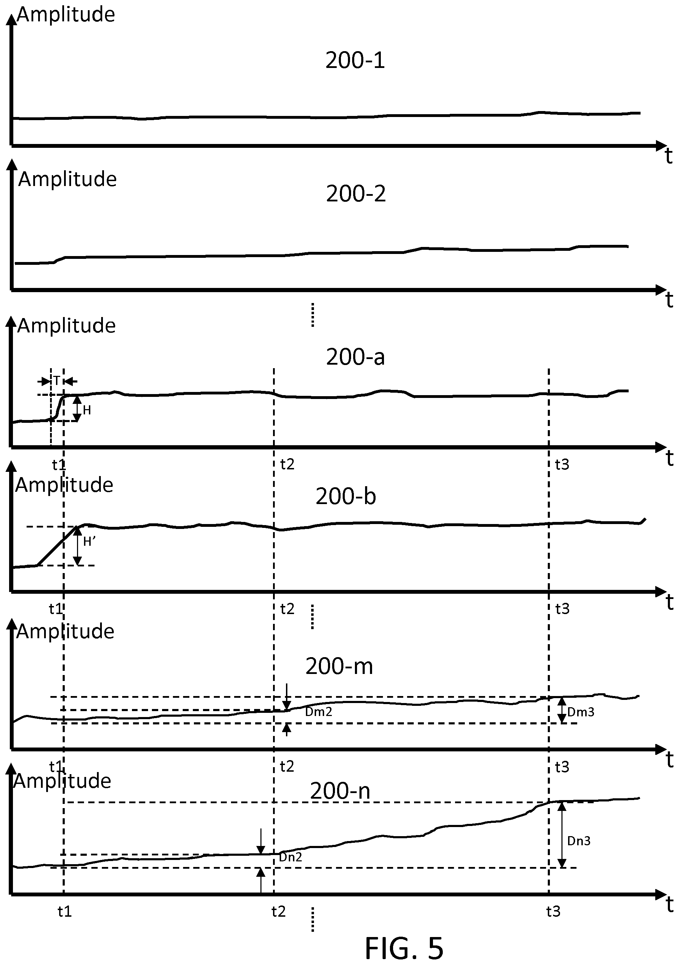

By using bandpass filter at the sense electrode that is configured to allow only a specific frequency to pass through and to filter out all other frequencies, a sense signal may be obtained for each frequency. Figure 5 illustrates amplitude vs. time graphs G showing the sense signals Ss of various frequencies, according to an embodiment of the present disclosure.

As an example, in Figure 5 from up to down, the uppermost graph G1 illustrates the sense signal 200-1 of 5 kHz, the second uppermost graph G2 illustrates the sense signal 200-2 of 10 kHz, ... , a graph Ga illustrates the sense signal 200-a of 5a kHz, a graph Gb illustrates the sense signal 200-b of 5b kHz, ..., a graph Gm illustrates the sense signal 200-m of 5m kHz, a graph Gn illustrates the sense signal 200-n of 5n kHz, ..., that are sensed from the sense electrode in response to the drive signal 100 as illustrated in Figure 4 being applied to the drive electrode, where a, b, m, and n are integers.

In the embodiment as illustrated in Figure 5, a first wetness event is assumed to occur at time t1, which causes the connection between the electrodes (the drive electrode and the sense electrode) in the absorbent article. In response to the first wetness event, a sharp jump in amplitude that changes beyond a threshold height Hj within a period shorter than a threshold period Tj may occur at or around t1 in a sense signal of a low frequency. This sense signal of the low frequency does not change in its amplitude much before and after this jump, even though further wetness events occur in the absorbent article later. It can be seen from Figure 5 that a jump in amplitude of a height H within a period T appears around t1 in signal 200-a and the signal 200-a does not change in its amplitude much before and after this jump, as an example.

Based on the above, in an embodiment of the present disclosure, a sharp jump in amplitude that changes beyond a threshold height Hj within a period shorter than a threshold period Tj in a sense signal of a low frequency can be used to determine the first wetness event in an absorbent article.

It is to be noted that, the change in amplitude of the sense signal of a low frequency in response to the first wetness event might be smoother, e.g. a gradual upslope, In some circumstances, in which case a threshold height Hs greater than the threshold height Hj may be used to determine the first wetness event in an absorbent article. That is, in addition or alternatively to the sharp jump in amplitude as described above, a gradual upslope in amplitude with a height beyond a threshold height Hs in a sense signal of a low frequency may be used to determine the first wetness event in an absorbent article, in an embodiment of the present disclosure. As an example, in Figure 5 an upslope in amplitude of a height H' occurs around t1 in the sense signal 200-b that also does not change much before and after the first wetness event t1.

Based on the above, in an embodiment of the present disclosure, a periodic drive signal that is swept in a period over a range of frequencies is applied to a drive electrode in an absorbent article, and by using bandpass filter, a sense signal is sensed from a sense electrode in the absorbent article for each frequency in the frequency range. In order to determine the first wetness event, the sense signals of low frequencies e.g. around 10 kHz are checked for a sharp jump e.g. of a height beyond Hj within a period T shorter than Tj or for a gradual slop e.g. of a height beyond Hs in their amplitudes.

As mentioned above, the sense signal of low frequency, with which the first wetness event in an absorbent article is determined by checking a sharp jump or a gradual upslope in its amplitude, does not change in amplitude much after the sharp jump or the gradual upslope, and thus cannot be further used to estimate or detect the saturation in the absorbent article.

On the other hand, a sense signal of high frequency e.g. ranging from 70 kHz - 500 Khz gradually increases in its amplitude as more and more wetness events occur in the absorbent article. Based on this, the sense signals of higher frequencies may be used to estimate or detect the saturation in the absorbent article after the first wetness event is determined, in an embodiment of the present disclosure.

It is understood that, after the first wetness event, further wetness events occur in the absorbent article. As further wetness events occur in the absorbent article, the sense signals of higher frequencies continue to increase in their amplitude gradually. In an embodiment of the present disclosure, in order to estimate or detect the saturation in the absorbent article, the difference in amplitude from the first wetness event (e.g. time t1 in Figure 5) is determined in each of all sense signals of higher frequencies. Once the difference in amplitude from the first wetness event (e.g. time t1 in Figure 5) is determined in any one of the sense signals of higher frequencies to be beyond a threshold amount Ts, the saturation is detected in the absorbent article.

As can be seen from Figure 5, at time t2 the difference in amplitude from the first wetness event t1 is Dm2 in the sense signal 200-m, and is Dn2 in the sense signal 200-n. However, the differences in amplitude from t1 to t2 are all less than the threshold amount Ts in all the sense signals of higher frequencies.

Further, at time t3, the difference in amplitude from the first wetness event t1 is Dm3 in the sense signal 200-m, and is Dn3 in the sense signal 200-n. At this time, while the differences in amplitude from t1 to t2 are less than the threshold amount Ts in other sense signals of higher frequencies, the difference in amplitude of the sense signal 200-n from t1 to t2 is greater than the threshold amount Ts, which may be used as indication of the saturation in the absorbent article, according to an embodiment of the present disclosure. In an embodiment of the present disclosure, the sense signal 200n, in which a difference in amplitude from t1 greater than the threshold amount Ts is first detected, may be e.g. about 100 kHz.

Based on above, in an embodiment of the present disclosure, a periodic drive signal that is swept in a period over a range of frequencies is applied to a drive electrode in an absorbent article, and by using bandpass filter, a sense signal is sensed from a sense electrode in the absorbent article for each frequency in the frequency range. In order to estimate or detect the saturation in the absorbent article, the first wetness event, which is determined as described above with use of a sense signal of low frequency, is used as baseline, and the difference in amplitude from the first wetness event is compared to a threshold amount Ts in each of all sense signals of higher frequencies e.g. ranging from 70 kHz to 500 kHz, and whenever a difference in amplitude is determined to be greater than the threshold amount Ts, the saturation is detected in the absorbent article.

It is also appreciated that, the wetness spot changes in different orientations of the user wearing the absorbent article, which in turn results in different distances between the wetness spot and the pod. Therefore, in an embodiment of the present disclosure, the threshold amount Ts may be changed depending on the orientation of the user. For example, according to an embodiment of the present disclosure, a smaller threshold amount Ts is assigned to an orientation of user in which the wetness spot is further away from the pod.

As described above, in an embodiment of the present disclosure, multiple frequencies are used for moisture detection and estimation of an absorbent article. In particular, a low frequency is used to determine the first wetness event in the absorbent article, which is in turn used as a baseline for moisture or saturation estimation, while another high frequency is used to estimate or detect the saturation of the absorbent article. As mentioned above, low frequencies are checked for a sharp jump or a gradual upslope in amplitude that acts as indication of the first wetness event in the absorbent article. Then the differences in amplitude from the baseline of high frequencies are compared to a threshold amount in order to detect or estimate the saturation of the absorbent article.

## Claims

1. A method of moisture detection and estimation in an absorbent article with use of multiple frequencies, comprising:
applying, by capacitive coupling, drive signal of multiple frequencies to a drive electrode in the absorbent article;
sensing, by capacitive coupling, sense signal of the multiple frequencies from a sense electrode in the absorbent article;
detecting the first wetness event in the absorbent article with use of sense signal of a frequency of the multiple frequencies; and
detecting the saturation of the absorbent article with use of sense signal of another frequency of the multiple frequencies.

2. The method according to Claim 1, wherein the detecting the first wetness event comprises:
detecting the first wetness event in the absorbent article when a sharp jump in amplitude is determined in the sense signal of the frequency that changes beyond a threshold height within a period shorter than a threshold period.

3. The method according to Claim 1, wherein the detecting the first wetness event comprises:
detecting the first wetness event in the absorbent article when a gradual upslope in amplitude is determined in the sense signal of the frequency that changes beyond a threshold height.

4. The method according to Claim 1, wherein the detecting the saturation comprises:
determining in the sense signal of the other frequency a difference in amplitude from the first wetness event; and
detecting the saturation when the difference in amplitude is greater than a threshold amount.

5. The method according to Claim 4, wherein the threshold amount is changed depending on the orientation of the absorbent article.

6. The method according to any of Claims 1 - 5, wherein the applying drive signal of multiple frequencies comprising:
applying a periodic drive signal that is swept in a period over the multiple frequencies.

7. The method according to any of Claims 1 - 5, wherein the sensing sense signal of the multiple frequencies comprises:
determining, for each of the multiple frequencies, sense signal by using a bandpass filter.

## Patentansprüche

1. Verfahren zur Feuchtigkeitserkennung und -schätzung in einem absorptionsfähigen Artikel unter Verwendung mehrerer Frequenzen, umfassend:
Anwenden eines Ansteuersignals mit mehreren Frequenzen auf eine Ansteuerelektrode in dem absorptionsfähigen Artikel durch kapazitive Kopplung;
Erfassen eines Erfassungssignals mit den mehreren Frequenzen von einer Erfassungselektrode in dem absorptionsfähigen Artikel durch kapazitive Kopplung;
Erkennen des ersten Nässeereignisses in dem absorptionsfähigen Artikel unter Verwendung eines Erfassungssignals mit einer Frequenz von den mehreren Frequenzen; und
Erkennen der Sättigung des absorptionsfähigen Artikels unter Verwendung eines Erfassungssignals mit einer anderen Frequenz von den mehreren Frequenzen.

2. Verfahren nach Anspruch 1, wobei das Erkennen des ersten Nässeereignisses umfasst:
Erkennen des ersten Nässeereignisses in dem absorptionsfähigen Artikel, wenn ein deutlicher Sprung einer Amplitude in dem Erfassungssignal mit der Frequenz bestimmt wird, der sich innerhalb einer Periode, die kürzer als eine Grenzperiode ist, über eine Grenzhöhe hinaus ändert.

3. Verfahren nach Anspruch 1, wobei das Erkennen des ersten Nässeereignisses umfasst:
Erkennen des ersten Nässeereignisses in dem absorptionsfähigen Artikel, wenn ein allmählicher Anstieg einer Amplitude in dem Erfassungssignal mit der Frequenz bestimmt wird, der sich über eine Grenzhöhe hinaus ändert.

4. Verfahren nach Anspruch 1, wobei das Erkennen der Sättigung umfasst:
Bestimmen eines Unterschieds einer Amplitude von dem ersten Nässeereignis in dem Erfassungssignal mit der anderen Frequenz; und
Erkennen der Sättigung, wenn der Unterschied der Amplitude größer als ein Grenzbetrag ist.

5. Verfahren nach Anspruch 4, wobei der Grenzbetrag in Abhängigkeit von der Ausrichtung des absorptionsfähigen Artikels geändert wird.

6. Verfahren nach einem der Ansprüche 1-5, wobei das Anwenden eines Ansteuersignals mit mehreren Frequenzen umfasst:
Anwenden eines periodischen Ansteuersignals, das in einer Periode über die mehreren Frequenzen gewobbelt wird.

7. Verfahren nach einem der Ansprüche 1-5, wobei das Erfassen eines Erfassungssignals mit den mehreren Frequenzen umfasst:
Bestimmen eines Erfassungssignals durch Verwenden eines Bandpassfilters für jede der mehreren Frequenzen.

## Revendications

1. Un procédé de détection et d'estimation de l'humidité dans un article absorbant à l'aide de multiples fréquences, comprenant :
appliquer, par couplage capacitif, un signal d'entraînement de multiples fréquences à une électrode d'entraînement dans l'article absorbant ;
détecter, par couplage capacitif, un signal de détection desdites multiples fréquences à partir d'une électrode de détection dans l'article absorbant ;
détecter le premier événement d'humidité dans l'article absorbant à l'aide d'un signal de détection d'une fréquence parmi les multiples fréquences ; et
détecter la saturation de l'article absorbant à l'aide d'un signal de détection d'une autre fréquence parmi les multiples fréquences.

2. Le procédé selon la revendication 1, dans lequel la détection du premier événement d'humidité comprend :
détecter un premier événement d'humidité dans l'article absorbant lorsqu'un saut brusque d'amplitude est détecté dans le signal de détection à la fréquence considérée, ladite amplitude dépassant une hauteur seuil dans une période inférieure à une période seuil.

3. Le procédé selon la revendication 1, dans lequel la détection du premier événement d'humidité comprend :
détecter le premier événement d'humidité dans l'article absorbant lorsqu'une augmentation progressive de l'amplitude est déterminée dans le signal de détection de la fréquence qui change au-delà d'une hauteur seuil.

4. Le procédé selon la revendication 1, dans lequel la détection de la saturation comprend :
déterminer dans le signal de détection de l'autre fréquence une différence d'amplitude par rapport au premier événement d'humidité ; et
détecter la saturation lorsque la différence d'amplitude est supérieure à une valeur seuil.

5. Le procédé selon la revendication 4, dans lequel la valeur seuil est modifiée en fonction de l'orientation de l'article absorbant.

6. Le procédé selon l'une quelconque des revendications 1 à 5, dans lequel l'application d'un signal d'entraînement de multiples fréquences comprend :
appliquer un signal d'entraînement périodique qui est balayé dans une période sur les multiples fréquences.

7. Le procédé selon l'une quelconque des revendications 1 à 5, dans lequel le signal de détection des multiples fréquences comprend :
déterminer, pour chacune des multiples fréquences, le signal de détection en utilisant un filtre passe-bande.
